# EUROPEAN PATENT SPECIFICATION

(11) **EP 4 076 153 B1**
(45) Date of publication and mention of the grant of the patent: **08.10.2025**
(21) Application number: 20838904.9
(22) Date of filing: 11.12.2020
(51) Int. Cl.: A61B 5/00, A61B 5/029, A61B 5/021

(54) **AORTIC STENOSIS CLASSIFICATION**
KLASSIFIZIERUNG VON AORTENSTENOSEN
CLASSIFICATION DE STÉNOSE AORTIQUE

(30) Priority: 17.12.2019 US 201962949037 P
(43) Date of publication of application: 26.10.2022
(73) Proprietor: Koninklijke Philips N.V., 5656 AG Eindhoven (NL)
(72) Inventor: SUN, Deyu, 5656 AE Eindhoven (NL); MARKUZON, Natalya, 5656 AE Eindhoven (NL)
(74) Representative: Philips Intellectual Property & Standards
(86) International application number: PCT/EP2020/085662
(87) International publication number: WO 2021/122345

(56) References cited:
- WO-A1-2019/153039
- WO-A1-2020/157212
- US-A1- 2018 107 787
- BAUMGARTNER HELMUT ET AL: "Recommendations on the Echocardiographic Assessment of Aortic Valve Stenosis: A Focused Update from the European Association of Cardiovascular Imaging and the American Society of Echocardiography", JOURNAL OF THE AMERICAN SOCIETY OF ECHOCARDIOGRAPHY, MOSBY-YEAR BOOK, INC. ST. LOUIS, MO, US, vol. 30, no. 4, 3 April 2017 (2017-04-03), pages 372 - 392, XP029966343, ISSN: 0894-7317, DOI: 10.1016/J.ECHO.2017.02.009

## Description

### FIELD OF THE INVENTION

The following generally relates to classification and more particularly to aortic stenosis classification.

### BACKGROUND OF THE INVENTION

Aortic stenosis (AS) occurs in almost 10% of adults over age 80 years with a mortality about 50% at 2 years unless outflow obstruction is relieved by aortic valve replacement, e.g. transcatheter aortic valve replacement (TAVR). The "AHA/ACC Guideline for the Management of Patients with Valvular Heart Disease" indicates that severe AS is defined by an aortic valve area (AVA) <1.0 cm², a mean transaortic pressure gradient (MPG) >40 mm Hg, and a peak aortic jet velocity (Vmax) >4 m/s. This guideline has been used for interventional planning. Unfortunately, not all severe AS patients meet the guideline. As a consequence, clinicians are sometimes in a grey area when diagnosing such patients, leading to an inconsistent assessment of AS severity with lack of guidance on the timing of intervention. In addition, potential factors that influence AS progression and their significance thereon are not well understood, and current approaches do not provide an accurate estimation of significance or progression rate.

The assessment of stenosis severity is described in WO-2019/153039-A1, US-2018/107787-A1, WO-2020/157212-A1, and BAUMGARTNER HELMUT ET AL: "Recommendations on the Echocardiographic Assessment of Aortic Valve Stenosis: A Focused Update from the European Association of Cardiovascular Imaging and the American Society of Echocardiography", JOURNAL OF THE AMERICAN SOCIETY OF ECHOCARDIOGRAPHY, vol. 30, no. 4, April 2017, ISSN: 0894-7317, DOI: 10.1016/J.ECHO.2017.02.009.

### SUMMARY OF THE INVENTION

Aspects described herein address the above-referenced problems and/or others.

The invention is defined by the claims.

In one aspect of the disclosure, a system includes a digital information repository(s) configured to store an aortic valve area measurement, a mean transaortic pressure gradient measurement, and a peak aortic jet velocity measurement for a subject of interest. The system further includes a computing apparatus. The computing apparatus comprises a memory configured to store instructions for an aortic stenosis classifier. The computing apparatus further comprises a processor configured to execute the stored instructions for the aortic stenosis classifier to classify a severity of an aortic stenosis of the subject of interest based at least on the aortic valve area measurement, the mean transaortic pressure gradient measurement, and the peak aortic jet velocity measurement for the subject of interest. The computing apparatus further comprises a display configured to display the severity.

In another aspect, a method includes obtaining information about a subject, including at least an aortic valve area measurement, a mean transaortic pressure gradient measurement, and a peak aortic jet velocity measurement for the subject, from a digital information repository. The method further includes obtaining instructions for an aortic stenosis classifier. The method further includes executing the instructions to classify a severity of an aortic stenosis of the subject of interest based at least on the aortic valve area measurement, the mean transaortic pressure gradient measurement, and the peak aortic jet velocity measurement for the subject of interest. The method further includes visually presenting the classified severity.

In another aspect, a computer-readable storage medium stores instructions that when executed by a processor of a computer cause the processor to: obtain information about a subject, including at least an aortic valve area measurement, a mean transaortic pressure gradient measurement, and a peak aortic jet velocity measurement for the subject, from a digital information repository, obtain instructions for an aortic stenosis classifier, and execute the instructions to classify a severity of an aortic stenosis of the subject of interest based at least on the aortic valve area measurement, the mean transaortic pressure gradient measurement, the peak aortic jet velocity measurement for the subject of interest, and visually present the classified severity.

Those skilled in the art will recognize still other aspects of the present application upon reading and understanding the attached description.

### BRIEF DESCRIPTION OF THE DRAWINGS

The invention may take form in various components and arrangements of components, and in various steps and arrangements of steps. The drawings are only for purposes of illustrating the embodiments and are not to be construed as limiting the invention.
FIG. 1 diagrammatically illustrates an example system configured for aortic stenosis classification, classification visualization, risk factor determination, and/or severity prediction, in accordance with an embodiment(s) herein.
FIG. 2 diagrammatically illustrates an example of the aortic stenosis classifier, in accordance with an embodiment(s) herein.
FIG. 3 diagrammatically illustrates example training of the aortic stenosis classifier, in accordance with an embodiment(s) herein.
FIG. 4 diagrammatically illustrates further example training of the aortic stenosis classifier, in accordance with an embodiment(s) herein.
FIG. 5 diagrammatically illustrates an example of the classification AS visualization engine, in accordance with an embodiment(s) herein.
FIG. 6 graphically shows example display of individual-level classification in two-dimensions, in accordance with an embodiment(s) herein.
FIG. 7 graphically shows example display of population-level data classification in three-dimensions, in accordance with an embodiment(s) herein.
FIG. 8 graphically shows a two-dimensional view of the population-level classification of FIG. 7, in accordance with an embodiment(s) herein.
FIG. 9 graphically shows another two-dimensional view of the population-level classification of FIG. 7, in accordance with an embodiment(s) herein.
FIG. 10 diagrammatically illustrates an example of the AS factor identifier, in accordance with an embodiment(s) herein.
FIG. 11 diagrammatically illustrates an example of the AS severity predictor, in accordance with an embodiment(s) herein.
FIG. 12 diagrammatically illustrates a variation of the aortic stenosis classifier of FIG. 1, in accordance with an embodiment(s) herein.
FIG. 13 illustrates an example method, in accordance with an embodiment(s) herein.
FIG. 14 illustrates another example method, in accordance with an embodiment(s) herein.
FIG. 15 illustrates yet another example method, in accordance with an embodiment(s) herein.
FIG. 16 illustrates still another example method, in accordance with an embodiment(s) herein.

### DETAILED DESCRIPTION OF EMBODIMENTS

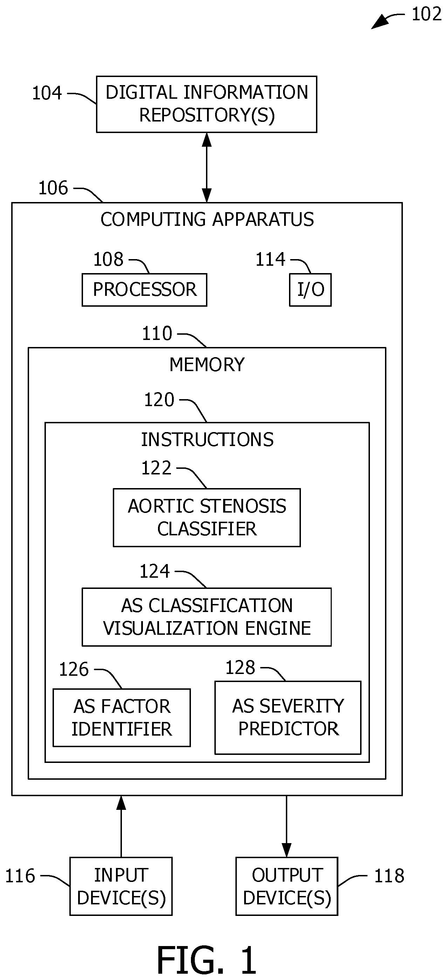

FIG. 1 diagrammatically illustrates an example system 102. The system 102 includes a digital information repository(s) 104 and a computing apparatus 106 (e.g., a computer or the like). The digital information repository(s) 104 includes a physical storage medium that stores digital information. This includes local, remote, distributed, and/or other physical storage medium. In one instance, the digital information repository(s) 104 includes at least information for subjects diagnosed with aortic stenosis, including historical aortic stenosis diagnosis, measurements (e.g., AVA, MPG and Vmax), and/or other information.

The illustrated computing apparatus 106 includes a processor 108 (e.g., a central processing unit (CPU), a microprocessor (µCPU), and/or other processor) and computer readable storage medium ("memory") 110 (which excludes transitory medium) such as a physical storage device like a hard disk drive, a solid-state drive, an optical disk, and/or the like. The processor 108 is configured to execute the instructions. Input/output ("I/O") 114 is configured for communication between the computing apparatus 106 and the digital information repository(s) 104, including receiving data from and/or transmitting a signal to the digital information repository(s) 104.

A human readable output device(s) 118, such as a display, is in electrical communication with the computing apparatus 106. In one instance, the human readable output device(s) 118 is a separate device configured to communicate with the computing apparatus 106 through a wireless and/or a wire-based interface. In another instance, the human readable output device(s) 118 is part of the computing apparatus 106. An input device(s) 116, such as a keyboard, mouse, a touchscreen, etc., is also in electrical communication with the computing apparatus 106.

The memory 110 includes instructions 120 at least for an aortic stenosis (AS) classifier 122, a AS classification visualization engine 124, an AS risk factor identifier 126, and/or AS severity predictor 128. As described in greater detail below, in one example, the aortic stenosis classifier 122 classifies aortic stenosis for a subject(s), the AS classification visualization engine 124 causes visualization of such classification, the AS risk factor identifier 126 identifies risk factors related to the progression of AS, and/or the AS severity predictor 128 predicts a severity of AS for the subject(s) based on the identified risk factors and/or provides information to the aortic stenosis classifier 122. In one instance, this provides information for a more accurate assessment of AS severity and/or a progression thereof, relative to a configuration in which the instructions 120 are not utilized and/or absent.

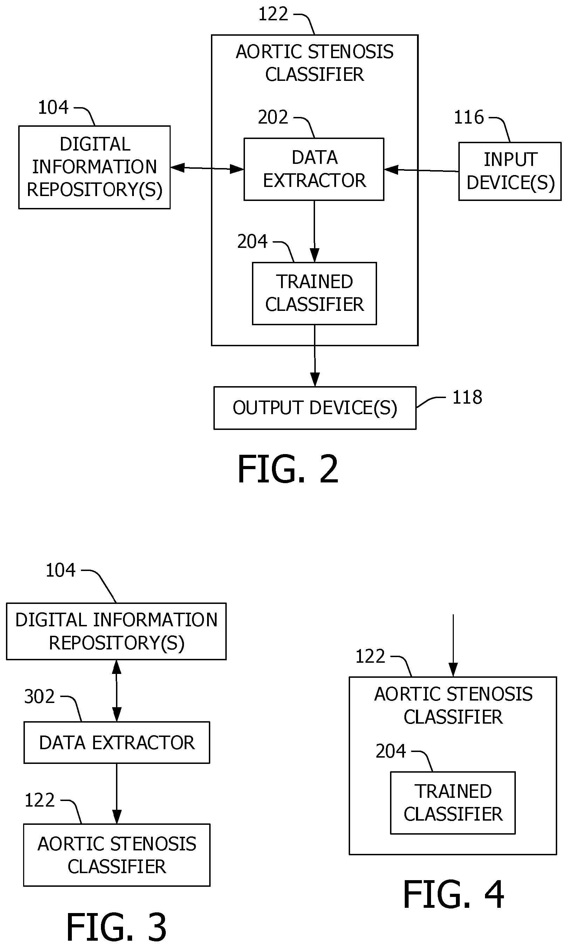

FIG. 2 diagrammatically illustrates an example of the aortic stenosis classifier 122. A data extractor 202 receives a subject identification (ID) of a subject of interest, e.g., from the input device(s) 116. The data extractor 202 extracts parameters for the subject of interest relevant to classifying a severity of an aortic stenosis of the subject of interest, e.g., AVA, MPG, and Vmax measurements, from the digital information repository(s) 104. A trained classifier 204 receives the extracted parameters. The trained classifier 204 classifies the aortic stenosis of the subject of interest based on the received extracted parameters. In this example, the aortic stenosis classifier 204 causes the display of the classification via a display monitor of the output device(s) 118.

In one instance, the trained classifier 204 includes a machine learning algorithm(s) such as linear regression, logistic regression, KNN classification, Support Vector Machine (SVM), decision trees, random forest, artificial neural network, K-means clustering, naive Bayes theorem, Recurrent Neural Networks (RNN) algorithm, and/or other machine leaming and/or other artificial intelligence algorithm(s). In one example, the trained classifier 204 utilizes majority voting based on multiple classifiers. By using majority voting, the trained classifier 204, in one instance, can provide better performance, e.g., when evaluating different classification problems, relative to an algorithm not including majority voting.

FIG. 3 diagrammatically illustrates example training of the aortic stenosis classifier 122. A data extractor 302 extracts parameters for a subject's diagnosed aortic stenosis from the digital information repository(s) 104. The data extractor 302 can be part of the instructions 112 of the computing apparatus 106 and/or other instructions of a different computing apparatus. In one instance, the parameters are extracted from a single healthcare entity / site. In another instance, the parameters are extracted from multiple healthcare entities / sites.

In this example, the extracted parameters represent a training set of data that includes historical aortic stenosis diagnosis of subjects in the digital information repository(s) 104, including at least the AVA, MPG, and Vmax measurements, fed to the aortic stenosis classifier 122. In one instance, the aortic stenosis classifier 122 is configured to determine relations between variables in making a solution, such as decision tree, logistic regression, etc. The aortic stenosis classifier 122 will identify values for each variable and/or combinations of values for variables that lead to each one of the classification values.

FIG. 4 diagrammatically illustrates further example training of the aortic stenosis classifier 122. In this example, the input data and classification results for a particular subject are provided to the aortic stenosis classifier 122 and used to dynamically update the trained classifier 204. In one instance, the trained classifier 204 is dynamically updated as classification results become available. In another instance, the trained classifier 204 is dynamically updated with classification results on demand. In yet another instance, the trained classifier 204 is dynamically updated with classification results based on a schedule.

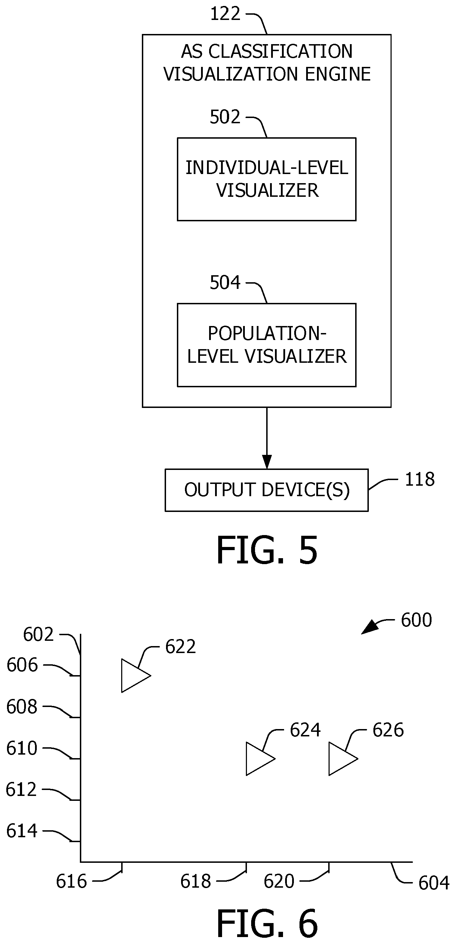

FIG. 5 diagrammatically illustrates an example of the AS classification visualization engine 124. The illustrated AS classification visualization engine 124 includes an individual-level visualizer 502 and/or a population-level visualizer 504. The AS classification visualization engine 124 causes display of information to assist clinicians in checking the parameters of a subject of interest. This information includes historical diagnoses of the subject of interest and hemodynamic parameters of the subject of interest and other subjects with aortic stenosis. In one instance, this allows a clinician to visually observe similar cases to assist diagnosis.

FIG. 6 graphically shows an example display of individual-level data 600 generated by the individual-level visualizer 502 of FIG. 5 in two-dimensions. In this example, the individual level data 600 includes historical diagnosis of a subject. A first axis 602 represents severity and a second axis 604 represents time. In this example, the first axis 602 includes: a mild severity level 606, a mild to moderate severity level 608, a moderate severity level 610, a moderate to severe severity level 612 and a severe severity level 614, and the second axis 604 indicates dates 616, 618 and 620 on which severity levels 622, 624 and 626 were determined. This display represents the progress of the disease and, in one instance, assists clinicians with understanding how fast and how severe the AS develops.

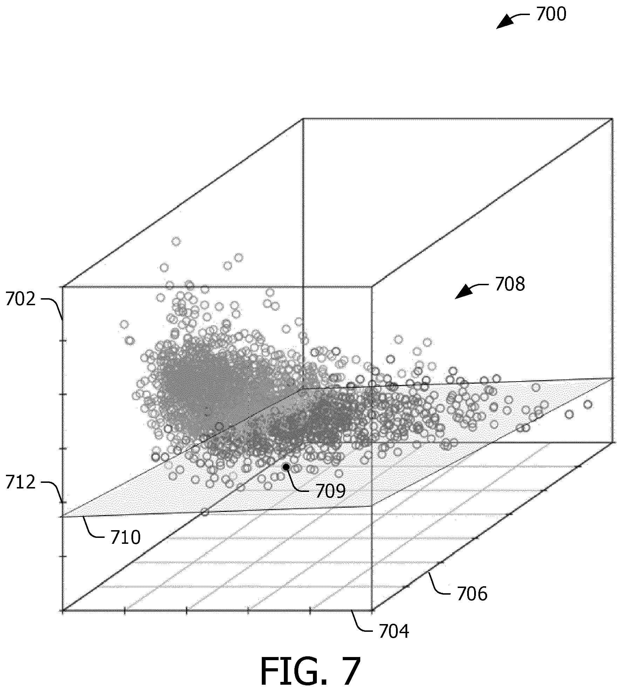

FIG. 7 graphically shows an example display of population-level data 700 generated by the population-level visualizer 504 of FIG. 5 in three-dimensions. A first axis 702 represents AVA, a second axis 704 represents MPG, and a third axis 706 represents Vmax. Each circle 708 represents a data point for each of the population subjects. A circle 709 represents a data point for the subject of interest. An AVA threshold plane 710 represents an aortic stenosis threshold, where the circles 708 under the AVA threshold plane 510 represent subjects likely with severe aortic stenosis and the circles 708 above the AVA threshold plane 510 represent subjects likely with non-severe aortic stenosis. In FIG. 7, a gray level shading is used with the circles 708 and 709 to distinguish between levels of severity with respect to the AVA threshold plane 510.

In this example, the AVA threshold plane 710 is not a horizontal plane at 1 cm², as recommended by the guideline. That is, the four corners of the AVA threshold plane 710 do not intersect the vertical axes at the same point. On the first axis, a level 712 represents the guideline value of 1 cm². In another instance, the AVA threshold plane 710 is a horizontal plane. In this instance, the AVA threshold plane 710 is at the guideline value of 1 cm². In another instance, it is not at the guideline value of 1 cm². The population-level data 700 visually assists clinicians with understanding the distribution of the three parameters (AVA, MPG and Vmax) and the classified groups with respect to the whole population.

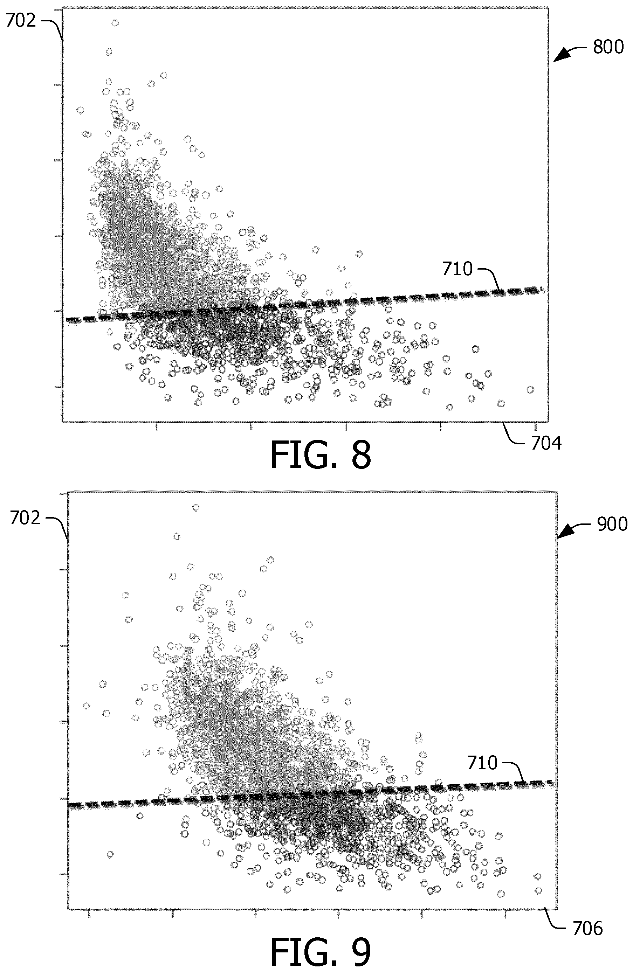

FIG. 8 graphically shows a two-dimensional view 800 of two axes of the population-level data 700 of FIG. 7, showing the first axis 702 (AVA) and the second axis 704 (MPG). The AVA threshold plane 710 is shown in two dimensions.

FIG. 9 graphically shows another two-dimensional view 900 of a different combination of two axes of the population level data 700 of FIG. 7, showing the first axis 702 (AVA) and the third axis 706 (Vmax). The AVA threshold plane 710 is shown in two dimensions. In another instance, the graphical display shows the second axis 704 (MPG) and the third axis 706 (Vmax) in two dimensions.

The above examples are described with particular application to aortic stenosis. However, it is to be understood that the above can be utilized for other diseases. For example, the approach described herein also applies to cardiovascular diseases that are diagnosed based only on cutoffs of a series of parameters, e.g., mitral regurgitation, mitral stenosis, etc. The degree of mitral regurgitation depends on, e.g., the volume of blood that flow through the mitral valve, aortic valve and the regurgitant orifice area, etc. The degree of mitral stenosis depends on, e.g., mean gradient, mitral valve area, etc.

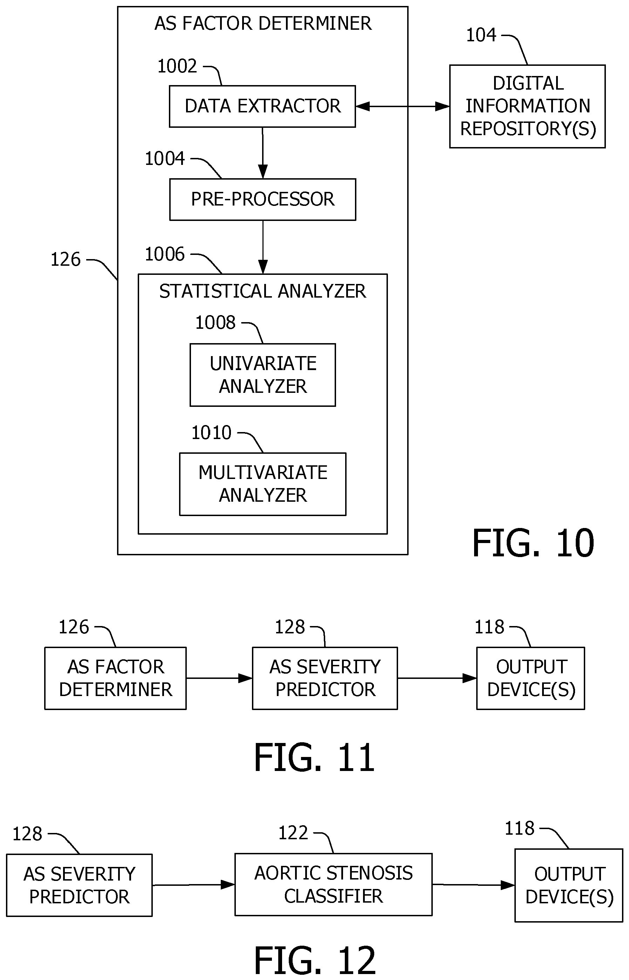

FIG. 10 diagrammatically illustrates an example of the AS factor determiner 126 in connection with the digital information repository(s) 104. In this example, the AS factor determiner 126 includes a data extractor 1002, a pre-processor 1004 and a statistical analyzer 1006.

The data extractor 1002 is configured to extract certain data from the digital information repository(s) 104. For example, in one instance the data extractor 1002 is configured to include information for subjects who have been subject to an adult transthoracic echocardiogram (TTE) and diagnosed with at least mild AS and exclude information for subjects that have prosthetic valves. The included information includes hemodynamic parameters measured by echocardiogram (i.e. AVA, NPG, Vmax, etc.), findings made by echo cardiologists, lab test results, vital signs, medications, problem lists, demographics, clinical notes, and/or other information.

The pre-processor 1004 is configured to pre-process the data extracted from the digital information repository(s) 104 with the data extractor 1002. This can be based on rules and/or otherwise. In one instance, this includes removing outliers. An example of an outlier includes erroneous data, e.g., due to recording and/or other errors. Taking AVA, for example, since a subject with AS cannot get better without surgery, if an AVA measurement has a more than a 15% increase comparing with the previous measurement, this measurement is considered an outlier and removed, discarded, ignored, and/or otherwise not utilized.

Additionally, or alternatively, this includes imputing missing information. This includes utilizing an algorithm tailored to the particular missing data. Additionally, or alternatively, this includes representing repeated data. This also includes utilizing an algorithm tailored to the repeated data. For example, where multiple temperatures taken at different points in time, the median and/or other value of a specified time window can be used to represent the temperature. Measurements like temperature, blood pressure, etc. are commonly measured repetitively. In one instance, natural language processing is used to extract features from information represented in free-text such as free-text discharge summary, clinical notes, etc.

The statistical analyzer 1006 includes a univariate analyzer 1008 and a multivariate analyzer 1010. The univariate analyzer 1008 is configured to employ a statistical model on each of the features from the pre-processor 1004. In one instance, this includes analyzing, for each factor, its interaction with time interval by a Linear Mixed-Effects (LME) model. For example, in one instance a LME model is used to consider a random intercept and a random slope. In this instance, if a feature is significantly associated with the hemodynamic parameters AVA, MPEG and Vmax (e.g., significance level = 0.05), the feature is provided to the multivariate analyzer 1010. Otherwise, it is not. The multivariate analyzer 1010 is configured to employ a statistical model on the features from the univariate analyzer 1008. In one instance, an LME model is used to evaluate the significance of the interaction terms of each feature and the time interval. Features considered significant features (e.g., significance level = 0.05) output as risk factors.

Table 1 below shows an example for ΔAVA. In this example, age, gender, left ventricular (LV) systolic function, and AVA calculated by the continuity equation were extracted for subgroup analysis. The AVA from subsequent echocardiograms of the same patient are compared to determine an annual rate of AVA change (ΔAVA). To estimate the annual AVA progression rate for each risk factor, the LME model includes the risk factor and the time interval between the measurements. The fixed effects include the risk factor, the time interval and their interaction term. The random effects include the time interval and a random intercept in order to account for the differing baseline AVA and progression rates due to individual characteristics that are not explained by the risk factor.

The significance of the risk factor upon the progression rate, in one instance, is analyzed from the p-value of the interaction term in the fixed effects. A median age at the time of the initial echocardiogram was 75 years, and 44% were male. A rate of progression of AS was -0.062±0.003 cm²/year. This rate was not influenced by age or LV function (P-value > 0.05). However, the rate was influenced by gender (P-value < 0.05) being more rapid in men compared to women. There is an inverse relationship between initial severity of AS and progression rate. The information in Table 1 provides clinical information on the expected interval before intervention is needed for severe AS.

**Table 1. Risk Factor Identification.**

| | | n | ΔAVA (cm²/year) | |
|---|---|---|---|---|
| | | | Annual rate of change (β) | P-value |
| All subjects | | 916 | -0.062±0.003 | |
| Age | Age ≥75yr | 468 | -0.057±0.004 | 0.08 |
| | Age <75yr | 448 | -0.066±0.004 | |
| Gender | Male | 404 | -0.068±0.004 | 0.03 |
| | Female | 512 | -0.057±0.003 | |
| AVA | Mild (>1.5cm²) | 292 | -0.082±0.004 (*) | <0.001 (* vs ^) |
| | Moderate (1.0-1.5cm²) | 466 | -0.057±0.003 (^) | |
| | Severe (<1.0cm²) | 158 | -0.023±0.008 (+) | <0.001 (^ vs +) |
| LV Function | Normal | 773 | -0.061±0.003 | 0.29 |
| | Reduced | 143 | -0.069±0.008 | |

FIG. 11 diagrammatically illustrates an example of the AS severity predictor 128 in connection with the AS factor determiner 126 and the output device(s) 118. The AS severity predictor 12, in this example, utilizes an LME model for each of the hemodynamic parameters AVA, MPG and Vmax. The following provides an example for AVA that can also be used for MPG and/or Vmax. In Table 1, since all the risk factors are categorical, the p-value using student's t-test is used to determine statistical significance of the progression rate, compared to the reference level of each risk factor.

Using this LME model, by imputing the time interval, the three hemodynamic parameters can be predicted in the future. In one instance, a predictive performance of a Logistic Regression classifier predicting severity of the AS is utilized using unseen test data. Given the hemodynamic parameters AVA, MPG and Vmax, the accuracy of identifying severe versus non-severe AS was 93%. The area under the curve (AUC), sensitivity, specificity, positive predictive value, negative predictive value, f score of the classifier were 0.98, 0.94, 0.94, 0.91, 0.95, and 0.92, respectively.

FIG. 12 diagrammatically illustrates an example in which the output of the LME model for each of the hemodynamic parameters AVA, MPG and Vmax is provided to the aortic stenosis classifier 122 of FIG. 2 and utilized thereby, in addition or alternative to, the information extracted from the digital information repository(s) 104, as described above. For example, in one instance the aortic stenosis classifier 122 utilizes the output of the LME model, in addition or alternative to, the AVA, MPG and Vmax measurements from the digital information repository(s) 104 to classify the aortic stenosis of the subject of interest based thereon. Similar to above, the aortic stenosis classifier 122, in one example, causes the display of the classification via the output device(s) 118.

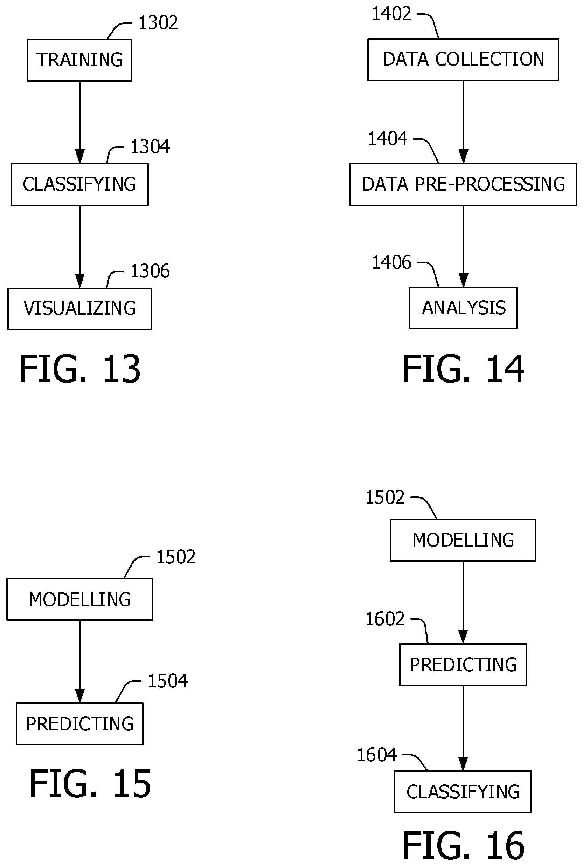

FIGS. 13, 14, 15 and 16 illustrate example methods in accordance with an embodiment(s) herein. It is to be appreciated that the ordering of the acts of one or more of the method is not limiting. As such, other orderings are contemplated herein. In addition, one or more acts may be omitted, and/or one or more additional acts may be included.

FIG. 13 illustrates an example method in accordance with an embodiment(s) herein. A training step 1302 trains a classifier, as described herein and/or otherwise. For example, in one instance the training step 1302 can train the classifier with AVA, MPG, Vmax and/or other information of subjects with aortic stenosis from the digital information repository(s) 104. A classifying step 1304 classifies aortic severity of a subject of interest, as described herein and/or otherwise. For example, in one instance classifying step 1304 classifies aortic severity of a subject of interest based on at least with AVA, MPG, Vmax of the subject of interest. A visualizing step 1306 visualizes the classification, as described herein and/or otherwise. For example, in one instance the visualizing step 1306 constructs and displays two and/or three dimensional graphs of aortic stenosis progression and/or AVA, MPG and/or Vmax.

FIG. 14 illustrates another example method in accordance with an embodiment(s) herein. A data collection step 1402 extracts certain data, as described herein and/or otherwise. For example, in one instance the data collection step 1402 extracts hemodynamic parameters of subjects who have had an adult transthoracic echocardiogram (TTE) and was diagnosed with at least mild AS, the parameters including AVA, NPG and Vmax, findings made by echo cardiologists, lab test results, vital signs, medications, problem lists, demographics, clinical notes, and/or other information.

A data pre-processing step 1404 pre-processes the extracted data, as described herein and/or otherwise. For example, in one instance the data pre-processing step 1404 at least one of removes outliers, imputes missing information, represents repeated measurements, or extracts from free text. An analysis step 1406 analyzes the pre-processed extracted data to determine a set of risk factors, as described herein and/or otherwise. For example, in one instance the analysis step 1406 filters the pre-processed extracted data via univariate analysis followed by multivariate analysis to keep only the factors considered significant based on a predetermined threshold.

FIG. 15 illustrates yet another example method in accordance with an embodiment(s) herein. A modelling step 1502 models hemodynamic parameters based on the risk factors of FIG. 14, as described herein and/or otherwise. For example, in one instance, an LME model is built for each of the hemodynamic parameters AVA, MPG and Vmax. A predicting step 1504 predicts a severity of aortic stenosis of a subject based on the hemodynamic parameters, as described herein and/or otherwise.

FIG. 16 illustrates still another example method in accordance with an embodiment(s) herein. A modelling step 1602 models hemodynamic parameters based on the risk factors of FIG. 14, as described herein and/or otherwise. For example, in one instance, an LME model is built for each of the hemodynamic parameters AVA, MPG and Vmax. A predicting step 1604 predicts a severity of aortic stenosis of a subject based on the hemodynamic parameters, as described herein and/or otherwise. A classifying step 1604 classifies aortic severity, as described herein and/or otherwise. For example, the classification step of FIG. 13 can additionally include training the classifier with the modelled hemodynamic parameters and then classifying aortic stenosis severity.

One or more of the above may be implemented by way of computer readable instructions, encoded or embedded on computer readable storage medium, which, when executed by a computer processor(s), cause the processor(s) to carry out the described acts. Additionally, or alternatively, at least one of the computer readable instructions is carried out by a signal, carrier wave or other transitory medium, which is not computer readable storage medium.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

The word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system (102), comprising:
a digital information repository(s) (104) configured to store an aortic valve area measurement, a mean transaortic pressure gradient measurement, and a peak aortic jet velocity measurement for a subject of interest;
a computing apparatus (106), comprising:
a memory (110) configured to store instructions (120) for an aortic stenosis classifier (122); and
a processor (108) configured to:
- extract information from the digital information repository(s) for subjects with aortic stenoses that do not have a prosthetic valve;
- process the extracted information to at least one of remove outliers, impute missing information, represent repeated measurements, or extract free text;
- perform an analysis on the processed extracted data to determine a set of risk factors of aortic stenosis progression;
- model each of aortic valve area, mean transaortic pressure gradient and peak aortic jet velocity based on the set of risk factors and predict a severity of aortic stenosis of the subject of interest based on the models;
- execute the stored instructions for the aortic stenosis classifier to classify a severity of an aortic stenosis of the subject of interest based at least on the aortic valve area measurement, the mean transaortic pressure gradient measurement, and the peak aortic jet velocity measurement for the subject of interest and an output of the model for each of the aortic valve area, the mean transaortic pressure gradient, and the peak aortic jet velocity; and
a display configured to display the severity.

2. The system of claim 1, wherein the digital information repository(s) is further configured to store information about subjects with aortic stenoses, including at least aortic valve area measurements, mean transaortic pressure gradient measurements, and peak aortic jet velocity measurements thereof, wherein the aortic stenosis classifier is trained with at least the aortic valve area measurements, the mean transaortic pressure gradient measurements, and the peak aortic jet velocity measurements of the subjects with aortic stenoses to provide a trained classifier (204).

3. The system of claim 2, wherein the instructions further includes an individual-level visualizer, and the processor is further configured to execute the instructions for the individual-level visualizer to construct a two-dimensional graph of aortic stenosis versus time based on historical aortic stenosis diagnoses and cause the display monitor to display the two-dimensional graph.

4. The system of claim 2, wherein the instructions further includes a population-level visualizer, and the processor is further configured to:
execute the instructions for the population-level visualizer to construct a three-dimensional graph of aortic valve area versus mean transaortic pressure gradient versus and peak aortic jet velocity, including:
a data point for the aortic valve area measurement, the mean transaortic pressure gradient measurement, and the peak aortic jet velocity measurement for the subject of interest;
data points for the aortic valve area measurements, the mean transaortic pressure gradient measurements, and the peak aortic jet velocity measurements of the subjects with aortic stenoses; and
an aortic stenosis threshold plane identifying combinations of values of the aortic valve area, the mean transaortic pressure gradient and the peak aortic jet velocity that indicate severe aortic stenosis; and
cause the display monitor to display the three-dimensional graph.

5. The system of claim 4, wherein the processor is further configured to construct a two-dimensional graph including the aortic valve area and the mean transaortic pressure gradient and cause the display monitor to display the two-dimensional graph.

6. The system of claim 4, wherein the processor is further configured to construct a two-dimensional graph including the aortic valve area and the peak aortic jet velocity and cause the display monitor to display the two-dimensional graph.

7. The system of claim **1,** wherein the analysis includes performing a univariate analysis for each subject of the subjects to determine initial risk factors associated with aortic valve area, mean transaortic pressure gradient and peak aortic jet velocity, followed by a multivariate analysis of the initial risk factors to determine the set of risk factors associated with the aortic valve area, the mean transaortic pressure gradient and the peak aortic jet velocity.

8. A computer-implemented method, comprising:
obtaining information about a subject, including at least an aortic valve area measurement, a mean transaortic pressure gradient measurement, and a peak aortic jet velocity measurement for the subject,
from a digital information repository;
extracting information for subjects with aortic stenoses that do not have a prosthetic valve;
processing the extracted data to at least one of remove outliers, impute missing information, represent repeated measurements, or extract free text;
performing an analysis on the processed extracted data to determine a set of risk factors of aortic stenosis progression;
modelling each of aortic valve area, mean transaortic pressure gradient, and peak aortic jet velocity based on the set of risk factors and predicting a severity of aortic stenosis for the subject based on the models;
obtaining instructions for an aortic stenosis classifier;
executing the instructions to classify a severity of an aortic stenosis of the subject of interest based at least on the aortic valve area measurement, the mean transaortic pressure gradient measurement, and the peak aortic jet velocity measurement for the subject of interest and an output of the model for each of the aortic valve area, the mean transaortic pressure gradient, and the peak aortic jet velocity; and
visually presenting the classified severity.

9. The computer-implemented method of claim 8, further comprising:
extracting information about subjects with aortic stenoses, including at least aortic valve area measurements, mean transaortic pressure gradient measurements, and peak aortic jet velocity measurements; and
training the aortic stenosis classifier with at least the aortic valve area measurements, the mean transaortic pressure gradient measurements, and the peak aortic jet velocity measurements of the subjects with aortic stenoses.

10. The computer-implemented method of claim 9, further comprising:
constructing at least one of a two-dimensional graph of aortic stenosis versus time or a three-dimensional graph of three-dimensional graph of aortic valve area versus mean transaortic pressure gradient versus and peak aortic jet velocity; and
displaying the constructed the at least one of the two-dimensional graph or the three-dimensional graph.

11. A computer-readable storage medium storing computer executable instructions which when executed by a processor of a computer cause the processor to perform the method according to any one of claims 8 to 10.

## Patentansprüche

1. System (102), das Folgendes umfasst:
einen digitalen Informationsbestand (digitale Informationsbestände) (104), der (die) dazu konfiguriert ist (sind), eine Aortenklappenflächenmessung, eine Messung des mittleren transaortischen Druckgradienten und eine Messung der aortischen Spitzenstrahlgeschwindigkeit für ein Subjekt von Interesse zu speichern;
eine Recheneinrichtung (106), die Folgendes umfasst:
einen Speicher (110), der dazu konfiguriert ist, Anweisungen (120) für einen Aortenstenose-Klassifizierer (122) zu speichern; und
einen Prozessor (108), der dazu konfiguriert ist:
- Informationen aus dem digitalen Informationsbestand (den digitalen Informationsbeständen) für Subjekte mit Aortenstenosen, die keine Klappenprothese besitzen, zu extrahieren;
- die extrahierten Informationen zum mindestens einen von Entfernen von Ausreißern, Einberechnen fehlender Informationen, Darstellen wiederholter Messungen oder Extrahieren von freiem Text zu verarbeiten;
- eine Analyse an den verarbeiteten extrahierten Daten durchzuführen, um einen Satz von Risikofaktoren für ein Fortschreiten der Aortenstenose zu bestimmen;
- jedes der Aortenklappenfläche, des mittleren transaortischen Druckgradienten und der aortischen Spitzenstrahlgeschwindigkeit basierend auf dem Satz von Risikofaktoren zu modellieren, und basierend auf den Modellen einen Schweregrad der Aortenstenose des Subjekts von Interesse vorherzusagen;
- die gespeicherten Anweisungen für den Aortenstenose-Klassifizierer auszuführen, um einen Schweregrad einer Aortenstenose des Subjekts von Interesse mindestens basierend auf der Aortenklappenflächenfläche, der Messung des mittleren transaortischen Druckgradienten und der Messung der aortischen Spitzenstrahlgeschwindigkeit für das Subjekt von Interesse und einer Ausgabe des Modells für jedes der Aortenklappenfläche, des mittleren transaortischen Druckgradienten und der aortischen Spitzenstrahlgeschwindigkeit zu klassifizieren; und
eine Anzeige, die dazu konfiguriert ist, den Schweregrad anzuzeigen.

2. System nach Anspruch 1, wobei der Informationsbestand (die Informationsbestände) weiter dazu konfiguriert ist (sind), Informationen über Subjekte mit Aortenstenosen einschließlich mindestens Aortenklappenflächenmessungen, Messungen des mittleren transaortischen Druckgradienten und Messungen der aortischen Spitzenstrahlgeschwindigkeit davon zu speichern, wobei der Aortenstenose-Klassifizierer mit mindestens den Aortenklappenflächenmessungen, den Messungen des mittleren transaortischen Druckgradienten und den Messungen der aortischen Spitzenstrahlgeschwindigkeit der Subjekte mit Aortenstenosen trainiert ist, um einen trainierten Klassifizierer (204) bereitzustellen.

3. System nach Anspruch 2, wobei die Anweisungen weiter einen Visualisierer auf individueller Ebene beinhalten, und der Prozessor weiter dazu konfiguriert ist, die Anweisungen für den Visualisierer auf individueller Ebene auszuführen, um ein zweidimensionales Schaubild der Aortenstenose gegenüber der Zeit basierend auf historischen Aortenstenosediagnosen aufzubauen und zu veranlassen, dass der Anzeigemonitor das zweidimensionale Schaubild anzeigt.

4. System nach Anspruch 2, wobei die Anweisungen weiter einen Visualisierer auf Populationsebene beinhalten, und der Prozessor weiter dazu konfiguriert ist:
die Anweisungen für den Visualisierer auf Populationsebene auszuführen, um ein dreidimensionales Schaubild der Aortenklappenfläche gegenüber dem mittleren transaortischen Druckgradienten und der aortischen Spitzenstrahlgeschwindigkeit aufzubauen, das Folgendes beinhaltet:
einen Datenpunkt für die Aortenklappenflächenmessung, die Messung des mittleren transaortischen Druckgradienten und die Messung der aortischen Spitzenstrahlgeschwindigkeit für das Subjekt von Interesse;
Datenpunkte für die Aortenklappenflächenmessungen, die Messungen des mittleren transaortischen Druckgradienten und die Messungen der aortischen Spitzenstrahlgeschwindigkeit der Subjekte mit Aortenstenosen; und
eine Aortenstenose-Schwellenebene, die Kombinationen von Werten der Aortenklappenfläche, des mittleren transaortischen Druckgradienten und der aortischen Spitzenstrahlgeschwindigkeit identifiziert, die auf schwere Aortenstenose hinweisen; und
zu veranlassen, dass der Anzeigemonitor das dreidimensionale Schaubild anzeigt.

5. System nach Anspruch 4, wobei der Prozessor weiter dazu konfiguriert ist, ein zweidimensionales Schaubild aufzubauen, das die Aortenklappenfläche und den mittleren transaortischen Druckgradienten beinhaltet, und den Anzeigemonitor zu veranlassen, das zweidimensionale Schaubild anzuzeigen.

6. System nach Anspruch 4, wobei der Prozessor weiter dazu konfiguriert ist, ein zweidimensionales Schaubild aufzubauen, das die Aortenklappenfläche und die aortische Spitzenstrahlgeschwindigkeit beinhaltet, und den Anzeigemonitor zu veranlassen, das zweidimensionale Schaubild anzuzeigen.

7. System nach Anspruch 1, wobei die Analyse das Durchführen einer univariaten Analyse für jedes Subjekt der Subjekte beinhaltet, um anfängliche Risikofaktoren zu bestimmen, die der Aortenklappenfläche, dem mittleren transaortischen Druckgradienten und der aortischen Spitzenstrahlgeschwindigkeit zugeordnet sind, gefolgt von einer multivariaten Analyse der anfänglichen Risikofaktoren, um den Satz von Risikofaktoren zu bestimmen, die der Aortenklappenfläche, dem mittleren transaortischen Druckgradienten und der aortischen Spitzenstrahlgeschwindigkeit zugeordnet sind.

8. Computerimplementiertes Verfahren, das Folgendes umfasst:
Erhalten von Informationen über ein Subjekt, die mindestens eine Aortenklappenflächenmessung, eine Messung des mittleren transaortischen Druckgradienten und eine Messung der aortischen Spitzenstrahlgeschwindigkeit für das Subjekt beinhalten,
aus einem digitalen Informationsbestand;
Extrahieren von Informationen für Subjekte mit Aortenstenosen, die keine Klappenprothese besitzen;
Verarbeiten der extrahierten Daten, zum mindestens einen von Entfernen von Ausreißern, Einberechnen fehlender Informationen, Darstellen wiederholter Messungen oder Extrahieren von freiem Text;
Durchführen einer Analyse an den verarbeiteten extrahierten Daten, um einen Satz von Risikofaktoren für ein Fortschreiten der Aortenstenose zu bestimmen;
Modellieren jedes der Aortenklappenfläche, des mittleren transaortischen Druckgradienten und der aortischen Spitzenstrahlgeschwindigkeit basierend auf dem Satz von Risikofaktoren, und Vorhersagen eines Schweregrades der Aortenstenose für das Subjekt von Interesse basierend auf den Modellen;
Erhalten von Anweisungen für einen Aortenstenose-Klassifizierer;
Ausführen der Anweisungen, um einen Schweregrad einer Aortenstenose des Subjekts von Interesse mindestens basierend auf der Aortenklappenflächemessung, der Messung des mittleren transaortischen Druckgradienten und der Messung der aortischen Spitzenstrahlgeschwindigkeit für das Subjekt von Interesse und eine Ausgabe des Modells für jedes der Aortenklappenfläche, des mittleren transaortischen Druckgradienten und der aortischen Spitzenstrahlgeschwindigkeit zu klassifizieren; und
visuelles Darstellen des klassifizierten Schweregrades.

9. Computerimplementiertes Verfahren nach Anspruch 8, das weiter Folgendes umfasst:
Extrahieren von Informationen über Subjekte mit Aortenstenosen, die mindestens Aortenklappenflächenmessungen, Messungen des mittleren transaortischen Druckgradienten und Messungen der aortischen Spitzenstrahlgeschwindigkeit beinhalten; und
Trainieren des Aortenstenose-Klassifizierers mit mindestens den Aortenklappenflächenmessungen, den Messungen des mittleren transaortischen Druckgradienten und den Messungen der aortischen Spitzenstrahlgeschwindigkeit der Subjekte mit Aortenstenosen.

10. Computerimplementiertes Verfahren nach Anspruch 9, das weiter Folgendes umfasst:
Aufbauen mindestens eines von einem zweidimensionalen Schaubild der Aortenstenose gegenüber der Zeit oder einem dreidimensionalen Schaubild der Aortenklappenfläche gegenüber dem mittleren transaortischen Druckgradienten und der aortischen Spitzenstrahlgeschwindigkeit; und
Anzeigen des aufgebauten mindestens einen des zweidimensionalen Schaubilds oder des dreidimensionalen Schaubilds.

11. Computerlesbares Speichermedium, das computerausführbare Anweisungen speichert, die, wenn sie von einem Prozessor eines Computers ausgeführt werden, den Prozessor veranlassen, das Verfahren nach einem der Ansprüche 8 bis 10 durchzuführen.

## Revendications

1. Système (102), comprenant :
un ou plusieurs référentiels d'informations numériques (104) configurés pour stocker une mesure de surface de valve aortique, une mesure de gradient de pression transaortique moyen et une mesure de vitesse de jet aortique de pic pour un sujet d'intérêt ;
un appareil informatique (106), comprenant :
une mémoire (110) configurée pour stocker des instructions (120) pour un classificateur de sténose aortique (122) ; et
un processeur (108) configuré pour :
- extraire des informations des un ou plusieurs référentiels d'informations numériques pour des sujets atteints de sténoses aortiques qui ne présentent pas de valve prothétique ;
- traiter les informations extraites pour au moins l'une des opérations suivantes : supprimer des valeurs aberrantes, imputer des informations manquantes, représenter des mesures répétées ou extraire du texte libre ;
- réaliser une analyse sur les données extraites traitées pour déterminer un ensemble de facteurs de risque de progression de sténose aortique ;
- modéliser chacun de la surface de valve aortique, du gradient de pression transaortique moyen et de la vitesse de jet aortique de pic sur la base de l'ensemble de facteurs de risque et prédire une gravité de la sténose aortique du sujet d'intérêt sur la base des modèles ;
- exécuter les instructions stockées pour le classificateur de sténose aortique pour classer une gravité d'une sténose aortique du sujet d'intérêt sur la base au moins de la mesure de la surface de la valve aortique, de la mesure du gradient de pression transaortique moyen et de la mesure de la vitesse maximale du jet aortique pour le sujet d'intérêt et d'une sortie du modèle pour chacune des surfaces de valve aortique, du gradient de pression transaortique moyen et de la vitesse maximale du jet aortique ; et
un dispositif d'affichage configuré pour afficher la gravité.

2. Système selon la revendication 1, dans lequel le ou les référentiels d'informations numériques sont en outre configurés pour stocker des informations concernant des sujets présentant des sténoses aortiques, incluant au moins des mesures de surface de valve aortique, des mesures de gradient de pression transaortique moyen et des mesures de vitesse de jet aortique de pic de celles-ci, dans lequel le classificateur de sténose aortique est entraîné avec au moins les mesures de surface de valve aortique, les mesures de gradient de pression transaortique moyen et les mesures de vitesse de jet aortique de pic des sujets présentant des sténoses aortiques pour fournir un classificateur entraîné (204).

3. Système selon la revendication 2, dans lequel les instructions incluent en outre un visualiseur de niveau individuel et le processeur est en outre configuré pour exécuter les instructions pour que le visualiseur de niveau individuel construise un graphique bidimensionnel de la sténose aortique versus le temps sur la base de diagnostics de sténose aortique historiques et amène le moniteur d'affichage à afficher le graphique bidimensionnel.

4. Système selon la revendication 2, dans lequel les instructions incluent en outre un visualiseur de niveau de population et le processeur est en outre configuré pour :
exécuter les instructions du visualiseur de niveau de population pour construire un graphique tridimensionnel de la surface de valve aortique versus le gradient de pression transaortique moyen versus la vitesse de jet aortique de pic, incluant :
un point de données pour la mesure de surface de valve aortique, la mesure de gradient de pression transaortique moyen et la mesure de vitesse de jet aortique de pic pour le sujet d'intérêt ;
des points de données pour les mesures de surface de valve aortique, les mesures de gradient de pression transaortique moyen et les mesures de vitesse de jet aortique de pic des sujets présentant des sténoses aortiques ; et
un plan de seuil de sténose aortique identifiant des combinaisons de valeurs de la surface de valve aortique, du gradient de pression transaortique moyen et de la vitesse de jet aortique de pic qui indiquent une sténose aortique grave ; et
amener le dispositif d'affichage à afficher le graphique tridimensionnel.

5. Système selon la revendication 4, dans lequel le processeur est en outre configuré pour construire un graphique bidimensionnel incluant la surface de valve aortique et le gradient de pression transaortique moyen et amener le moniteur d'affichage à afficher le graphique bidimensionnel.

6. Système selon la revendication 4, dans lequel le processeur est en outre configuré pour construire un graphique bidimensionnel incluant la surface de valve aortique et la vitesse de jet aortique de pic et amener le moniteur d'affichage à afficher le graphique bidimensionnel.

7. Système selon la revendication 1, dans lequel l'analyse inclut la réalisation d'une analyse univariée pour chaque sujet des sujets pour déterminer des facteurs de risque initiaux associés à la surface de valve aortique, au gradient de pression transaortique moyen et à la vitesse de jet aortique de pic, suivie d'une analyse multivariée des facteurs de risque initiaux pour déterminer l'ensemble de facteurs de risque associés à la surface de valve aortique, au gradient de pression transaortique moyen et à la vitesse de jet aortique de pic.

8. Procédé mis en œuvre par ordinateur, comprenant :
l'obtention d'informations concernant un sujet, incluant au moins une mesure de surface de valve aortique, une mesure de gradient de pression transaortique moyen et une mesure de vitesse de jet aortique de pic pour le sujet,
à partir d'un référentiel d'informations numériques ;
l'extraction d'informations pour des sujets présentant des sténoses aortiques qui ne présentent pas de valve prothétique ;
le traitement des données extraites pour au moins l'une des opérations suivantes : supprimer des valeurs aberrantes, imputer des informations manquantes, représenter des mesures répétées ou extraire du texte libre ;
la réalisation d'une analyse sur les données extraites traitées pour déterminer un ensemble de facteurs de risque de progression de sténose aortique ;
la modélisation de chacun de la surface de valve aortique, du gradient de pression transaortique moyen et de la vitesse de jet aortique de pic sur la base de l'ensemble de facteurs de risque et la prédiction d'une gravité de la sténose aortique pour le sujet sur la base des modèles ;
l'obtention d'instructions pour un classificateur de sténose aortique ;
l'exécution des instructions stockées pour classer une gravité d'une sténose aortique du sujet d'intérêt sur la base au moins de la mesure de la surface de la valve aortique, de la mesure du gradient de pression transaortique moyen et de la mesure de la vitesse maximale du jet aortique pour le sujet d'intérêt et d'une sortie du modèle pour chacune des surfaces de valve aortique, du gradient de pression transaortique moyen et de la vitesse maximale du jet aortique ; et
la présentation visuelle de la gravité classée.

9. Procédé mis en œuvre par ordinateur selon la revendication 8, comprenant en outre :
l'extraction d'informations concernant des sujets présentant des sténoses aortiques, incluant au moins des mesures de surface de valve aortique, des mesures de gradient de pression transaortique moyen et des mesures de vitesse de jet aortique de pic ; et
l'entraînement du classificateur de sténose aortique avec au moins les mesures de surface de valve aortique, les mesures de gradient de pression transaortique moyen et les mesures de vitesse de jet aortique de pic des sujets présentant des sténoses aortiques.

10. Procédé mis en œuvre par ordinateur selon la revendication 9, comprenant en outre :
la construction d'au moins un d'un graphique bidimensionnel de la sténose aortique versus le temps ou d'un graphique tridimensionnel de la surface de valve aortique versus le gradient de pression transaortique moyen versus la vitesse de jet aortique de pic ; et
l'affichage du construit de l'au moins un du graphique bidimensionnel ou du graphique tridimensionnel.

11. Support de stockage lisible par ordinateur stockant des instructions exécutables par ordinateur qui, lorsqu'elles sont exécutées par un processeur d'un ordinateur, amènent le processeur à réaliser le procédé selon l'une quelconque des revendications 8 à 10.
